# FASCICULE DE BREVET EUROPEEN

(11) **EP 1 331 916 B1**
(45) Date de publication et mention de la délivrance du brevet: **13.08.2008**
(21) Numéro de dépôt: 01982579.3
(22) Date de dépôt: 26.10.2001
(51) Int. Cl.: A61K 8/04, A61K 8/92, A61Q 1/10

(54) **MASCARA COMPRENANT UNE PHASE GRASSE LIQUIDE ET UNE CIRE**
MASCARA, DIE EIN WACHS UND EINE FLÜSSIGE FETTPHASE ENTHÄLT
MASCARA COMPRISING A LIQUID FATTY PHASE AND A WAX

(30) Priorité: 27.10.2000 FR 0013868
(43) Date de publication de la demande: 06.08.2003
(73) Titulaire: L'ORÉAL, 75008 Paris (FR)
(72) Inventeur: COLLIN, Nathalie, F-92330 Sceaux (FR); PIOT, Bertrand, F-75009 Paris (FR)
(74) Mandataire: Boulard, Denis
(86) Numéro de dépôt international: PCT/FR2001/003351
(87) Numéro de publication internationale: WO 2002/034215

(56) Documents cités:
- EP-A- 0 005 922
- EP-A- 0 557 196
- EP-A- 1 013 256
- DE-U- 20 009 445
- DATABASE CHEMICAL ABSTRACTS [en ligne] STN; access number 126: 229 425, XP002174457 & JP 09 030924 A (SHISEIDO CO. LTD) 4 février 1997 (1997-02-04)
- Fiche technique Dow-Corning "Information about low viscosity silicone: 200 fluids 5, 10, 20 cs" 1997

## Description

La présente invention a pour objet une composition de revêtement des fibres kératiniques comprenant une cire et une phase grasse liquide. L'invention se rapporte également à un procédé de revêtement des fibres kératiniques. La composition et le procédé selon l'invention sont plus particulièrement destinés aux cils d'êtres humains, mais également aux faux-cils. La composition peut être une composition de maquillage des cils appelée aussi mascara, une base de maquillage des cils, une composition à appliquer sur un mascara, dite encore top-coat, ou bien encore une composition de traitement cosmétique des cils. Plus spécialement, l'invention porte sur un mascara.

Généralement, les compositions de revêtement des cils, appelées mascaras, comprennent des cires pour obtenir un gainage des cils. Ces cires peuvent être dispersées dans un milieu aqueux, notamment à l'aide de tensioactifs. Or le film de maquillage obtenu avec ces compositions a tendance à s'effriter dans le temps. Le film ainsi fragilisé à tendance à ne pas résister aux frottements, notamment des doigts et/ou à l'eau, lors de baignades ou de douches par exemple, ce qui est contraire à l'obtention d'un maquillage présentant une bonne tenue dans le temps.
Le document EP 0 557 196 décrit par exemple des mascaras à milieu aqueux comprenant des microdispersions de cires. Le document EP 1 013 256 décrit par exemple des mascaras comprenant une phase aqueuse dispersée dans une phase grasse liquide.

Il est aussi connu des mascaras résistants à l'eau, dit "waterproof", qui comprennent des cires dans un milieu anhydre, notamment dans une phase grasse liquide comprenant des solvants organiques. Ces compositions peuvent comprendre en outre une phase aqueuse dispersée dans la phase grasse liquide, comme le décrit par exemple le document WO-A-91/12793. Ce document précise par ailleurs que la résistance à l'eau du mascara peut être améliorée en ajoutant dans la phase aqueuse un polymère filmogène hydrosoluble. Néanmoins, avec ces mascaras , les cils maquillés sont peu épaissis et le maquillage obtenu est un maquillage naturel, peu chargeant. Si l'on veut obtenir un maquillage épais des cils, c'est-à-dire obtenir un mascara chargeant, il est nécessaire d'incorporer dans la phase grasse une quantité plus importantes de cire. Mais l'augmentation du taux de cire provoque une élévation de la viscosité du mascara ; ce dernier s'applique très difficilement sur les cils. Le mascara trop visqueux a un contact sec avec les cils, ne glisse pas sur les cils et freine l'application de la brosse sur les cils. Ces difficultés pour l'application d'une tel mascara ne permettent pas un dépôt correct sur les cils et le maquillage n'épaissit pas les cils.

Le but de la présente invention est d'éviter les inconvénients mentionnés précédemment et de disposer d'une composition de revêtement des fibres kératiniques, notamment des cils, s'appliquant facilement sur les matières kératiniques et conduisant, après application, à un revêtement résistant à l'eau qui épaissit les fibres kératiniques. La demanderesse a maintenant constaté de façon surprenante qu'un tel revêtement des fibres kératiniques, notamment des cils, pouvait être obtenu en utilisant une microdispersion aqueuse de particules de cire dans une composition comprenant une phase aqueuse et une phase grasse liquide. On obtient alors une composition qui s'applique facilement sur les fibres kératiniques et qui forme sur les fibres kératiniques un revêtement résistant à l'eau. Le maquillage obtenu épaissit bien les fibres kératiniques : on obtient un maquillage suffisamment chargeant. Par ailleurs, le revêtement ne s'effrite pas et présente une bonne résistance aux frottements, notamment aux frottements des doigts. De plus, lorsque la cire sous forme de microdispersion de cire est une cire dure telle que définie ci-après, la composition confère un recourbement satisfaisant des fibres kératiniques et le recourbement obtenu présente une bonne tenue dans le temps : le recourbement des cils est maintenu pendant au moins 6 heures.

De façon plus précise, la présente invention a pour objet une composition comprenant, dans un milieu physiologiquement acceptable, au moins une phase grasse liquide comprenant au moins un solvant organique volatil, et au moins une phase aqueuse, caractérisée par le fait que la phase aqueuse contient une microdispersion de particules de cire.

Un autre objet de l'invention est un produit de mascara comprenant un réservoir contenant une composition de mascara telle que définie précédemment, et muni d'un système d'application de la composition sur les fibres kératiniques, notamment les cils.

L'invention a aussi pour objet un procédé de revêtement des fibres kératiniques, notamment des cils, comprenant l'application sur les fibres kératiniques d'une composition telle que définie précédemment. Le procédé de revêtement des fibres kératiniques peut être un procédé de maquillage ou de traitement cosmétique non thérapeutique des fibres kératiniques, notamment des cils.

L'invention a également pour objet l'utilisation d'une composition telle que définie précédemment pour épaissir les fibres kératiniques, notamment les cils et/ou pour obtenir un maquillage chargeant des fibres kératiniques.

L'invention a encore pour objet l'utilisation, dans une composition comprenant un milieu physiologiquement acceptable, d'une microdispersion aqueuse de particules de cire, la cire ayant une dureté supérieure ou égale à 6 MPa, et d'une phase grasse liquide comprenant au moins un solvant organique volatil pour épaissir et/ou recourber les fibres kératiniques, notamment les cils.

L'invention a aussi pour objet l'utilisation, dans une composition comprenant un milieu physiologiquement acceptable, d'une microdispersion aqueuse de particules de cire, d'une phase grasse liquide comprenant au moins un solvant organique volatil, et d'une cire additionnelle présente dans la phase grasse liquide, pour épaissir les fibres kératiniques, notamment les cils et/ou obtenir un maquillage chargeant des fibres kératiniques.

L'invention a encore pour objet l'utilisation, dans une composition comprenant un milieu physiologiquement acceptable, d'une microdispersion aqueuse de particules de cire, la cire ayant une dureté supérieure ou égale à 6 MPa, d'une phase grasse liquide comprenant au moins un solvant organique volatil, et d'une cire additionnelle présente dans la phase grasse liquide, pour épaissir et/ou recourber les fibres kératiniques, notamment les cils.

Dans la présente demande, on entend par "composition de revêtement des fibres kératiniques" une composition apte à former un film sur les fibres kératiniques.

Par physiologiquement acceptable, il faut comprendre un milieu compatible avec les matières kératiniques, comme un milieu cosmétique.

### a) la microdispersion de cire :

La composition selon l'invention comprend une microdispersion aqueuse de particules de cire. On entend par microdispersion aqueuse de cire, une dispersion aqueuse de particules de cire, dans laquelle la taille moyenne desdites particules de cire est inférieure ou égale à environ 1 µm.

On entend par « taille moyenne inférieure ou égale à 1 µm » la taille pour laquelle au moins 50 % en volume des particules ont une taille inférieure ou égale à la taille moyenne de 1 µm. La taille des particules de cire peut être mesurée à l'aide d'un granulomètre, par exemple le granulomètre commercialisé sous la référence Mastersizer 2000 par la société Malvern.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les microdispersions de cire sont des dispersions de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire. Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 1 µm), de préférence inférieures à 0,5 µm (notamment allant de 0,051 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées sous forme de microdispersion dans la composition selon l'invention sont choisies parmi les cires, solides et rigides, à température ambiante d'origine animale, végétale, minérale ou de synthèse et leurs mélanges. De préférence, les cires peuvent présenter un point de fusion supérieur ou égal à 45°C environ (notamment allant de 45°C à 120°C), et en particulier supérieur ou égal à 55°C (notamment allant de 55°C à 120°C), voire même supérieur ou égal à 70 °C (notamment allant de 70 °C à 120 °C). La cire peut également présenter une dureté allant de 0,05 MPa à 15 MPa. Avantageusement, on peut utiliser une cire (dite cire dure) ayant une dureté supérieure ou égale à 6 MPa, notamment allant de 6 MPa à 30 MPa, et en particulier allant de 6 MPa à 15 MPa, pour obtenir une composition conférant un bon recourbement aux fibres kératiniques. De plus, le recourbement obtenu présente une bonne tenue dans le temps : le recourbement des cils est maintenu pendant au moins 6 heures.

La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par à la surface du cylindre du texturomètre en contact avec la cire.

Comme cire ayant une dureté supérieure ou égal à 6 MPa (dite cire dure), on peut citer la cire de carnauba, la cire microcristalline, les cires de polyéthylène, la cire de Candelilla, l'huile de jojoba hydrogénée, la cire de son de riz, les Ozokérites, la Cire d'insecte de chine, la cire de Shellac, la cire d'Ouricury.

Comme cire ayant une dureté allant inférieure à 6 MPa (notamment supérieure ou égale à 0,05 MPa et inférieure à 6 MPa) (dite cire molle), on peut utiliser la cire d'abeille, l'huile de ricin hydrogénée, la cire de lanoline, la cire de paraffine, la cire de Bayberry.

La composition selon l'invention peut comprendre, de préférence, de 0,1 à 50% en poids de matière sèche de cire de ladite microdispersion de cire, notamment 1 à 30% en poids. Elle peut également comprendre une quantité suffisante de tensioactif pour permettre d'obtenir une microdispersion de cire, ainsi qu'une composition finale, stable. Notamment, elle peut comprendre 0,01 à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

II est possible d'introduire en outre dans la phase cireuse microparticulaire des ingrédients actifs liposolubles, tels que des filtres U.V., des vitamines liposolubles, des actifs cosmétiques liposolubles.

### b) la phase grasse liquide :

La phase grasse liquide de la composition comprend au moins un solvant organique volatil.

On entend par "phase grasse liquide" dans la présente invention, tout milieu non aqueux liquide à température ambiante et non miscible à l'eau.
On entend par "solvant organique volatil" un solvant organique susceptible de s'évaporer à température ambiante d'un support sur lequel il a été appliqué, autrement dit un solvant ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles facilitent, notamment, l'application de la composition sur les fibres kératiniques. Ces huiles peuvent être des huiles hydrocarbonées ou des huiles siliconées.

Selon un mode de réalisation de l'invention, le solvant organique volatil peut être une huile hydrocarbonée volatile. On entend par "huile hydrocarbonée", une huile ne contenant que des atomes d'hydrogène et de carbone.

Les huiles hydrocarbonées volatiles préférées convenant pour la composition selon l'invention sont en particulier les isoparaffines, à savoir des alcanes ramifiés, comportant de 8 à 16 atomes de carbone telles que les 'ISOPARs', les PERME-TYLs et notamment l'isododécane (encore appelé 2,2,4,4,6-pentaméthylheptane). On peut bien entendu également utiliser des mélanges de telles isoparaffines. D'autres huiles hydrocarbonées volatiles comme les distillats de pétrole, notamment ceux vendus sous la dénomination Shell Solt par la société SHELL, peuvent aussi être utilisés.

Le solvant organique volatil peut être présent dans la composition selon l'invention en une teneur allant de 35 % à 75 % en poids, par rapport au poids total de la composition, de préférence de 45 % à 70 % en poids, et mieux de 50 % à 65 % en poids.

Comme solvants organiques volatils on peut utiliser aussi les silicones volatiles, comme par exemple les huiles de silicones cycliques et volatiles telles que l'octaméthylcyclotétrasiloxane, le décaméthylcyclopentasiloxane, le hexadécaméthylcyclohexasiloxane, les silicones linéaires volatiles telles que l'octaméthyltrisiloxane, l'heptaméthylhexyltrisiloxane, l'heptaméthyloctyltrisiloxane, ou bien encore les huiles volatiles fluorées telles que le nonafluorométhoxybutane ou le perfluorométhylcyclopentane.

La phase grasse liquide peut également contenir des huiles non volatiles, et notamment des huiles hydrocarbonées et/ou siliconées et/ou fluorées non volatiles. Comme huile hydrocarbonée non volatile, on peut notament citer :
- les huiles hydrocarbonées d'origine animale telle que le perhydrosqualène;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité;
- les hydrocarbures linéaires ou ramifiés, d'origine minérale ou synthétique tels que la vaseline, les polydécènes, le polyisobutène hydrogéné tel que le parléam;
- les esters et les éthers de synthèse comme les huiles de formule R₁₀COOR₁₁ dans laquelle R₁₀ représente le reste d'un acide gras supérieur comportant de 6 à 29 atomes de carbone et R₁₁ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle ; les esters de polyols comme le dioctanoate de propylène glycol, le diheptanoate de néopentylglycol, le diisonanoate de diéthylène glycol et les esters du pentaérythritol;
- les alcools gras liquides à température ambiante à chaîne carbonée ramifiée et/ou insaturée ayant de 12 à 26 atomes de carbone comme l'octyl dodécanol, l'alcool oléique, le 2-hexyldécanol, le 2-butyloctanol, le 2-undécylpentadécanol;
- les acides gras supérieurs tels que l'acide oléique, l'acide linoléique, l'acide lino-lénique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 5 % en poids, par rapport au poids total de la composition, de préférence de 0 % à 2 % en poids. Avantageusement, la composition selon l'invention ne contient pas d'huile non volatile, la composition permettant ainsi d'obtenir un très bon recourbement des cils.

Avantageusement, la phase aqueuse peut être dispersée dans la phase grasse liquide ; la composition se présente alors sous la forme d'émulsion eau-dans-huile ou huile-dans-eau, et de préférence sous la forme d'émulsion eau-dans-huile.

### c) les additifs :

La composition selon l'invention peut contenir, dans la phase grasse liquide, au moins une cire additionnelle. Cette cire additionnelle ne se présente pas sous la forme d'une microdispersion aqueuse de particules de cire telle que définie précédemment. La cire additionnelle permet d'obtenir un maquillage épais des cils. La composition comprenant la cire additionnelle peut donc être utilisée pour épaissir les fibres kératiniques, notamment les cils. Cette composition peut également être utilisée pour recourber les fibres kératiniques, notamment les cils, lorsque la cire en microdispersion est une cire dure telle que définie précédemment.

La cire additionnelle peut avoir une dureté allant de 0,05 MPa à 15 MPa. Elle peut être choisie parmi les cires citées précédemment (cires dures et cires molles).

La cire additionnelle contenue dans la phase grasse liquide peut être présente en une teneur allant de 5 % à 35 % en poids, par rapport au poids total de la composition, et de préférence de 15 % à 25 % en poids.

La composition selon l'invention peut contenir en outre, dans la phase grasse liquide, un polymère filmogène auxiliaire lipophile. Ce polymère filmogène auxiliaire lipophile peut notamment être solubilisé, ou dit encore liposoluble, ou dispersé, notamment en présence d'un stabilisant, dans la phase grasse liquide. Ce polymère filmogène lipophile confère notamment une bonne tenue du revêtement après application de la composition sur les fibres kératiniques.

Comme polymère lipophile, on peut notamment citer les copolymères résultant de la copolymérisation d'au moins un ester vinylique et d'au moins un autre monomère qui peut être une α-oléfine, un alkylvinyléther ou un ester allylique ou méthallylique, comme décrits dans la demande FR-A-2232303.

Comme polymères filmogènes lipophiles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Des dispersions de polymère filmogène dans la phase grasse liquide, en présence d'agent stabilisants, sont notamment décrites dans les documents EP-A-749746, EP-A-923928, EP-A-930060.

Le polymère filmogène auxiliaire lipophile de la phase grasse liquide peut être présent dans la composition en une teneur allant de 0,5 % à 15 % en poids, par rapport au poids total de la composition, et mieux de 2 % à 10 % en poids.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX. Ces argiles sont généralement associées, de façon connue, avec un activateur comme le carbonate de propylène ou l'éthanol pour obtenir l'épaississement de la phase grasse liquide.

L'agent épaississant peut être présent en une teneur allant de 0,5 % à 15 % en poids, par rapport au poids total de la composition, et mieux de 1 % à 10 % en poids.

Dans la composition selon l'invention, le poids total de la phase grasse liquide peut aller de 65 % à 99 % en poids, par rapport au poids total de la composition, et mieux de 80 % à 99 % en poids.

La composition peut également comprendre au moins une matière colorante comme les composés pulvérulents et/ou les colorants liposolubles, par exemple à raison de 0,01 à 30 % du poids total de la composition. Les composés pulvérulents peuvent être choisis parmi les pigments et/ou les nacres, autres que celles décrites précédemment, habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition selon l'invention peut également contenir des ingrédients couramment utilisés en cosmétique, tels que les agents plastifiants, les charges, les conservateurs, les agents hydratants, les parfums, les vitamines, les agents alcalinisants ou acidifiants habituellement utilisés dans le domaine cosmétique, et leurs mélanges

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée de façon connue pour l'homme du métier en faisant d'abord fondre les cires aditionnelles et en mélangeant les ingrédients de la phase grasse, y compris les additifs lipophiles. On ajoute ensuite les pigments et les charges, ainsi que les solvants préalablement épaissis. On prépare ensuite la phase aqueuse par mélange des constituants, on ajoute la microdispersion aqueuse de cire et on disperse la totalité de la phase aqueuse dans la phase grasse.

La composition selon l'invention est destiné à un produit de mascara comprenant un réservoir, contenant ladite composition de mascara, et un système d'application de ladite composition sur les fibres kératiniques, notamment les cils. Le réservoir est muni de façon connue d'une ouverture dans laquelle est logée un système d'essorage. Le système d'application comporte une tige munie à une première extrémité d'une brosse et à une deuxième extrémité d'un bouchon destiné à fermer le réservoir. Un tel conditionnement est notamment illustré à la figure 7 de la demande EP-A-611170.

L'invention est illustrée plus en détail dans les exemples suivants.

### Exemple 1 :

On a préparé une microdispersion de cire de carnauba ayant la composition suivante :

| | |
|---|---|
| - Cire de carnauba | 30 g |
| - Monostéarate de glycéryle polyoxyéthyléné (30 OE) | |
| (TAGAT S de GOLDSCHMIDT) | 7,5 g |
| - Conservateurs | 0,5 g |
| - Eau qsp | 100g |

On a chauffé à 95 °C la cire, le tensioactif et le conservateur en homogénéisant le mélange sous agitation modérée. Puis on a incorporé l'eau chauffée à 95 °C en continuant d'agiter. On a refroidit à température ambiante pour obtenir une microdispersion de cire ayant un diamètre moyen de particules d'environ 170 nm.

### Exemple 2 :

On a préparé un mascara ayant la composition suivante :

| | | |
|---|---|---|
| - Microdispersion de cire de l'exemple 1 | | 6, 6 g |
| - Cire de carnauba | | 5,4 g |
| - Cire de son de riz | | 2,55 g |
| - Paraffine | | 2,5 g |
| - Cire d'abeille | | 9,55 g |
| - Bentonite | | 5, 4 g |
| - Carbonate de propylène | | 1,7 g |
| - Alcool éthylique | | 0,7 g |
| - Copolymère acétate de vinyle/stéarate d'allyle (65/35) | | |
| (Mexomère PQ de CHIMEX) | | 7,2 g |
| - Polylaurate de vinyle (Mexomère PP de CHIMEX) | | 0,75 g |
| - D- panthénol | | 0,2g |
| - Talc | | 0,98 g |
| - Pigments | | 4,8 g |
| - Conservateurs | qs | |
| -Isododécane | qsp | 100 g |

Ce mascara s'applique facilement sur les cils et permet d'obtenir un maquillage résistant à l'eau et épaississant les cils. On obtient également un bon recourbement des cils qui est maintenu pendant au moins 6 heures.

## Revendications

1. Composition de revêtement des fibres kératiniques comprenant, dans un milieu physiologiquement acceptable, au moins une phase grasse liquide comprenant au moins un solvant organique volatil ayant une tension de vapeur, à pression et température ambiante allant de 10⁻³ à 300 mm de Hg (0,13 Pa à 40.000 Pa), à condition que la température d'ébullition soit supérieure à 30°C, et au moins une phase aqueuse, **caractérisée par le fait que** la phase aqueuse contient une microdispersion de particules de cire comprenant des particules de cires ayant une taille moyenne inférieure à 1 µm et en ce que ledit solvant organique est présent en une teneur allant de 35 % à 75 % en poids, par rapport au poids total de la composition.

2. Composition selon la revendication 1, **caractérisée par le fait que** la microdispersion de cires comprend des particules de cires ayant une taille moyenne inférieure à 0,5 µm.

3. Composition selon l'une quelconque des revendications précédentes, **caractérisée par le fait que** la cire est choisie dans le groupe formé par les cires ayant un point de fusion allant de 30 °C à 120 °C.

4. Composition selon l'une quelconque des revendications précédentes, **caractérisée par le fait que** la cire a une dureté supérieure ou égale à 6 MPa, de préférence allant de 6 MPa à 15 MPa.

5. Composition selon l'une des revendications précédentes, **caractérisée par le fait que** la cire est choisie dans le groupe formé par la cire de carnauba, la cire microcristalline, les cires de polyéthylène, la cire de Candelilla, l'huile de jojoba hydrogénée, la cire de son de riz, les Ozokérites, la Cire d'insecte de chine, la cire de Shellac, la cire d'Ouricury.

6. Composition selon l'une des revendications précédentes, **caractérisé par le fait que** la cire de ladite microdispersion est présente en une teneur en matière sèche allant de 0,1 % à 50 % en poids, par rapport au poids total de la composition, et mieux allant de 1 % à 30% en poids.

7. Composition selon l'une des revendications précédentes, dans laquelle les particules de la dispersion de cire comprennent en outre des additifs gras huileux et/ou pâteux et/ou un additif/actif liposoluble.

8. Composition selon l'une des revendications précédentes, comprenant en outre au moins un tensioactif.

9. Composition cosmétique selon l'une quelconque des revendications précédentes, **caractérisée par le fait que** le solvant organique volatil est choisi dans le groupe formé par les huiles hydrocarbo nées volatiles.

10. Composition cosmétique selon l'une quelconque des revendications précédentes, **caractérisée par le fait que** le solvant organique volatil est choisi dans le groupe formé par les isoparaffines comportant de 8 à 16 atomes de carbone.

11. Composition cosmétique selon l'une quelconque des revendications précédentes, **caractérisée par le fait que** le solvant organique volatil est présent en une teneur allant de 45 % à 70 % en poids par rapport au poids total de la composition, et mieux de 50 % à 65 % en poids.

12. Composition cosmétique selon l'une quelconque des revendications précédentes, **caractérisée par le fait que** la phase grasse liquide comprend au moins un polymère filmogène auxiliaire lipophile.

13. Composition selon la revendication 12, **caractérisée par le fait que** le polymère filmogène auxiliaire lipophile est présent en une teneur allant de 0,5 % à 15 % en poids, par rapport au poids total de la composition.

14. Composition selon l'une des revendications précédentes, comprenant en outre au moins un polymère filmogène additionnel solubilisé ou dispersé dans la phase aqueuse.

15. Composition selon l'une quelconque des revendications précédentes, **caractérisée par le fait que** la phase grasse liquide comprend une cire additionnelle.

16. Composition selon la revendication 15, **caractérisée par le fait que** la cire additionnelle a une dureté allant de 0,05 MPa à 15 MPa.

17. Composition selon la revendication 15. **caractérisée par le fait que** la cire additionnelle a une dureté supérieure ou égale à 0,05 MPa et inférieure à 6 MPa.

18. Composition selon la revendication 17, **caractérisée par le fait que** la cire additionnelle est choisie dans le groupe formé par la cire d'abeille, l'huile de ricin hydrogénée, la cire de lanoline, la cire de paraffine, la cire de Bayberry.

19. Composition selon l'une quelconque des revendications 15 à 18, **caractérisée par le fait que** la cire additionnelle est présente en une teneur allant de 5 % à 35 % en poids, par rapport au poids total de la composition, et de préférence de 15 % à 25 % en poids.

20. Composition selon l'une quelconque des revendications précédentes, **caractérisée par le fait que** la composition est une émulsion eau-dans-huile.

21. Composition selon l'une quelconque des revendications précédentes, **caractérisé par le fait que** la composition comprend, en outre, au moins un additif choisi dans le groupe formé par les épaississants, les agents plastifiants, les charges, les matières colorantes, les conservateurs, les agents hydratants, les parfums, les vitamines, les agents alcalinisants ou acidifiants, et leurs mélanges.

22. Composition selon l'une quelconque des revendications précédentes, **caractérisée par le fait que** la composition est une composition de revêtement des cils.

23. Composition selon l'une quelconque des revendications précédentes, **caractérisée par le fait que** la composition est un mascara.

24. Produit de mascara comprenant un réservoir, contenant une composition de mascara , et un système d'application de ladite composition sur les fibres kératiniques, **caractérisé par le fait que** la composition est une composition selon l'une quelconque des revendications 1 à 23.

25. Procédé cosmétique de revêtement des fibres kératiniques, notamment les cils, comprenant l'application sur lesdites fibres kératiniques d'une composition selon l'une quelconque des revendications 1 à 23.

26. Utilisation d'une composition selon l'une quelconque des revendications 1 à 23, pour recourber les fibres kératiniques, notamment les cils,

27. Utilisation d'une composition selon l'une quelconque des revendications 4 à 23, pour épaissir les fibres kératiniques, notamment les cils et/ou obtenir un maquillage chargeant des fibres kératiniques.

28. Utilisation, dans une composition comprenant un milieu physiologiquement acceptable, d'une microdispersion aqueuse de particules de cire comprenant des particules de cires ayant une taille moyenne inférieure à 1 µm, la cire ayant une dureté supérieure ou égale à 6 MPa, de préférence allant de 6 MPa à 15 MPa , et d'une phase grasse liquide comprenant au moins un solvant organique volatil ayant une tension de vapeur, à pression et température ambiante, allant de 10⁻³ à 300 mm de Hg (0,13 Pa à 40.000 Pa) à condition que la température d'ébullition soit supérieure à 30°C, pour épaissir et/ou recourber les fibres kératiniques, notamment les cils.

29. Utilisation, dans une composition comprenant un milieu physiologiquement acceptable, d'une microdispersion aqueuse de particules de cire comprenant des particules de cires ayant une taille moyenne inférieure à 1 µm, d'une phase grasse liquide comprenant au moins un solvant organique volatil ayant une tension de vapeur, à pression et température ambiante, allant de 10⁻³ à 300 mm de Hg (0,13 Pa à 40.000 Pa), à condition que la température d'ébullition soit supérieure à 30°C,, et d'une cire additionnelle presente dans la phase grasse liquide, pour épaissir les fibres kératiniques, notamment les cils.

30. Utilisation, dans une composition comprenant un milieu physiologiquement acceptable, d'une microdispersion aqueuse de particules de cire comprenant des particules de cires ayant une taille moyenne inférieure à 1 µm , la cire ayant une dureté supérieure ou égale à 6 MPa, de préférence allant de 6 MPa à 15 MPa , d'une phase grasse liquide comprenant au moins un solvant organique volatil ayant une tension de vapeur, à pression et température ambiante, allant de 10⁻³ à 300 mm de Hg (0,13 Pa à 40.000 Pa, à condition que la température d'ébullition soit supérieure à 30°C,, et d'une cire additionnelle présente dans la phase grasse liquide, pour épaissir et/ou recourber les fibres kératiniques, notamment les cils.

## Claims

1. Composition for coating keratinous fibres comprising, in a physiologically acceptable medium, at least one liquid fatty phase comprising at least one volatile organic solvent having a vapour pressure, at ambient pressure and temperature, ranging from 10⁻³ to 300 mmHg (0.13 Pa to 40,000 Pa), provided that the boiling point is greater than 30°C, and at least one aqueous phase, **characterized in that** the aqueous phase contains a microdispersion of particles of wax comprising particles of waxes having a mean size of less than 1 µm and **in that** the said organic solvent is present in an amount ranging from 35% to 75% by weight, relative to the total weight of the composition.

2. Composition according to Claim 1, **characterized in that** the microdispersion of waxes comprises particles of waxes having a mean size of less than 0.5 µm.

3. Composition according to either of the preceding claims, **characterized in that** the wax is chosen from the group formed by waxes having a melting point ranging from 30°C to 120°C.

4. Composition according to any one of the preceding claims, **characterized in that** the wax has a hardness greater than or equal to 6 MPa, preferably ranging from 6 MPa to 15 MPa.

5. Composition according to one of the preceding claims, **characterized in that** the wax is chosen from the group formed by carnauba wax, microcrystalline wax, polyethylene waxes, Candelilla wax, hydrogenated jojoba oil, rice bran wax, Ozokerites, Chinese wax, Shellac wax, Ouricury wax.

6. Composition according to one of the preceding claims, **characterized in that** the wax of the said microdispersion is present at a dry matter content ranging from 0.1% to 50% by weight, relative to the total weight of the composition, and better still from 1% to 30% by weight.

7. Composition according to one of the preceding claims, in which the particles of the wax dispersion comprise, in addition, oily and/or pasty fatty additives and/or a fat-soluble additive/active agent.

8. Composition according to one of the preceding claims, comprising, in addition, at least one surfactant.

9. Cosmetic composition according to any one of the preceding claims, **characterized in that** the volatile organic solvent is chosen from the group formed by volatile hydrocarbon oils.

10. Cosmetic composition according to any one of the preceding claims, **characterized in that** the volatile organic solvent is chosen from the group formed by isoparaffins comprising from 8 to 16 carbon atoms.

11. Cosmetic composition according to any one of the preceding claims, **characterized in that** the volatile organic solvent is present in an amount ranging from 45% to 70% by weight, relative to the total weight of the composition, and better still from 50% to 65% by weight.

12. Cosmetic composition according to any one of the preceding claims, **characterized in that** the liquid fatty phase comprises at least one lipophilic auxiliary film-forming polymer.

13. Composition according to Claim 12, **characterized in that** the lipophilic auxiliary film-forming polymer is present in an amount ranging from 0.5% to 15% by weight, relative to the total weight of the composition.

14. Composition according to one of the preceding claims, comprising, in addition, at least one additional film-forming polymer solubilized or dispersed in the aqueous phase.

15. Composition according to any one of the preceding claims, **characterized in that** the liquid fatty phase comprises an additional wax.

16. Composition according to Claim 15, **characterized in that** the additional wax has a hardness ranging from 0.05 MPa to 15 MPa.

17. Composition according to Claim 15, **characterized in that** the additional wax has a hardness greater than or equal to 0.05 MPa and less than 6 MPa.

18. Composition according to Claim 17, **characterized in that** the additional wax is chosen from the group formed by beeswax, hydrogenated castor oil, lanolin wax, paraffin wax and Bayberry wax.

19. Composition according to any one of Claims 15 to 18, **characterized in that** the additional wax is present in an amount ranging from 5% to 35% by weight, relative to the total weight of the composition, and preferably from 15% to 25% by weight.

20. Composition according to any one of the preceding claims, **characterized in that** the composition is a water-in-oil emulsion.

21. Composition according to any one of the preceding claims, **characterized in that** the composition comprises, in addition, at least one additive chosen from the group formed by thickeners, plasticizers, fillers, colouring matter, preservatives, moisturizers, perfumes, vitamins, alkalinizing or acidifying agents, and mixtures thereof.

22. Composition according to any one of the preceding claims, **characterized in that** the composition is a composition for coating the eyelashes.

23. Composition according to any one of the preceding claims, **characterized in that** the composition is a mascara.

24. Mascara product comprising a reservoir, containing a mascara composition, and a system for applying the said composition to the keratinous fibres, **characterized in that** the composition is a composition according to any one of Claims 1 to 23.

25. Cosmetic method for coating the keratinous fibres, in particular the eyelashes, comprising the application to the said keratinous fibres of a composition according to any one of Claims 1 to 23.

26. Use of a composition according to any one of Claims 1 to 23, for curling the keratinous fibres, in particular the eyelashes.

27. Use of a composition according to any one of Claims 4 to 23, for thickening the keratinous fibres, in particular the eyelashes, and/or for obtaining a high-load make-up application to the keratinous fibres.

28. Use, in a composition comprising a physiologically acceptable medium, of an aqueous microdispersion of particles of wax comprising particles of wax having a mean size of less than 1 µm, the wax having a hardness greater than or equal to 6 MPa, preferably ranging from 6 MPa to 15 MPa, and a liquid fatty phase comprising at least one volatile organic solvent having a vapour pressure, at ambient pressure and temperature, ranging from 10⁻³ to 300 mmHg (0.13 Pa to 40,000 Pa), provided that the boiling point is greater than 30°C, for thickening and/or curling the keratinous fibres, in particular the eyelashes.

29. Use, in a composition comprising a physiologically acceptable medium, of an aqueous microdispersion of particles of wax comprising particles of wax having a mean size of less than 1 µm, a liquid fatty phase comprising at least one volatile organic solvent having a vapour pressure, at ambient pressure and temperature, ranging from 10⁻³ to 300 mmHg (0.13 Pa to 40,000 Pa), provided that the boiling point is greater than 30°C, and an additional wax present in the liquid fatty phase, for thickening the keratinous fibres, in particular the eyelashes.

30. Use, in a composition comprising a physiologically acceptable medium, of an aqueous microdispersion of particles of wax comprising particles of wax having a mean size of less than 1 µm, the wax having a hardness greater than or equal to 6 MPa, preferably ranging from 6 MPa to 15 MPa, a liquid fatty phase comprising at least one volatile organic solvent having a vapour pressure, at ambient pressure and temperature, ranging from 10⁻³ to 300 mmHg (0.13 Pa to 40,000 Pa), provided that the boiling point is greater than 30°C, and an additional wax present in the liquid fatty phase, for thickening and/or curling the keratinous fibres, in particular the eyelashes.

## Patentansprüche

1. Zusammensetzung zum Überziehen von Keratinfasern, die in einem physiologisch akzeptablen Medium mindestens eine flüssige Fettphase, die mindestens ein flüchtiges organisches Lösungsmittel aufweist, das bei Umgebungsdruck und Raumtemperatur einen Dampfdruck im Bereich von 10⁻³ bis 300 mm Hg (0,13 Pa bis 40 000 Pa) besitzt, mit der Maßgabe, dass die Siedetemperatur über 30 °C liegt, und mindestens eine wässrige Phase enthält, **dadurch gekennzeichnet, dass** die wässrige Phase eine Mikrodispersion von Wachspartikeln enthält, die Wachspartikel mit einer mittleren Größe unter 1 µm umfasst, und **dadurch**, dass das organische Lösungsmittel in einer Menge von 35 bis 75 Gew.-%, bezogen auf das Gesamtgewicht der Zusammensetzung, enthalten ist.

2. Zusammensetzung nach Anspruch 1, **dadurch gekennzeichnet, dass** die Wachsmikrodispersion Wachspartikel mit einer mittleren Größe unter 0,5 µm umfasst.

3. Zusammensetzung nach einem der vorhergehenden Ansprüche, **dadurch gekennzeichnet, dass** das Wachs unter den Wachsen mit einem Schmelzpunkt von 30 bis 120 °C ausgewählt ist.

4. Zusammensetzung nach einem der vorhergehenden Ansprüche, **dadurch gekennzeichnet, dass** das Wachs eine Härte größer oder gleich 6 MPa und vorzugsweise im Bereich von 6 bis 15 MPa aufweist.

5. Zusammensetzung nach einem der vorhergehenden Ansprüche, **dadurch gekennzeichnet, dass** das Wachs unter Carnaubawachs, mikrokristallinen Wachsen, Polyethylenwachsen, Candelillawachs, hydriertem Jojobaöl, Reiskleiewachs, Ozokeriten, Chinawachs, Schellackarachs und Ouricurywachs ausgewählt ist.

6. Zusammensetzung nach einem der vorhergehenden Ansprüche, **dadurch gekennzeichnet, dass** das Wachs der Mikrodispersion in einer Trockensubstanzmenge von 0,1 bis 50 Gew.-%, bezogen auf das Gesamtgewicht der Zusammensetzung und besser im Bereich von 1 bis 30 Gew.-% enthalten ist.

7. Zusammensetzung nach einem der vorhergehenden Ansprüche,
**dadurch gekennzeichnet, dass** die Partikel der Wachsdispersion ferner ölige und/oder pastöse Fettadditive und/oder einen fettlöslichen Zusatzstoff/Wirkstoff enthalten.

8. Zusammensetzung nach einem der vorhergehenden Ansprüche, die ferner mindestens einen grenzflächenaktiven Stoff enthält.

9. Kosmetische Zusammensetzung nach einem der vorhergehenden Ansprüche, **dadurch gekennzeichnet, dass** das flüchtige organische Lösungsmittel unter den flüchtigen Kohlenwasserstoffölen ausgewählt ist,

10. Kosmetische Zusammensetzung nach einem der vorhergehenden Ansprüche, **dadurch gekennzeichnet, dass** das flüchtige organische Lösungsmittel unter den Isoparaffinen mit 8 bis 16 Kohlenstoffatomen ausgewählt ist.

11. Kosmetische Zusammensetzung nach einem der vorhergehenden Ansprüche, **dadurch gekennzeichnet, dass** das flüchtige organische Lösungsmittel in einer Menge von 45 bis 70 Gew.-%, bezogen auf das Gesamtgewicht der Zusammensetzung, und besser 50 bis 65 Gew.-% vorliegt.

12. Kosmetische Zusammensetzung nach einem der vorhergehenden Ansprüche, **dadurch gekennzeichnet, dass** die flüssige Fettphase mindestens ein ergänzendes lipophiles filmbildendes Polymer enthält.

13. Zusammensetzung nach Anspruch 12, **dadurch gekennzeichnet, dass** das ergänzende lipophile filmbildende Polymer in einer Menge von 0,5 bis 15 Gew.-%, bezogen auf das Gesamtgewicht der Zusammensetzung, enthalten ist.

14. Zusammensetzung nach einem der vorhergehenden Ansprüche, die ferner mindestens ein ergänzendes filmbildendes Polymer enthält, das in der wässrigen Phase solubilisiert oder dispergiert ist.

15. Zusammensetzung nach einem der vorhergehenden Ansprüche; **dadurch gekennzeichnet, dass** die flüssige Fettphase ein ergänzendes Wachs enthält.

16. Zusammensetzung nach Anspruch 15, **dadurch gekennzeichnet, dass** das ergänzende Wachs eine Härte von 0,05 bis 15 MPa aufweist.

17. Zusammensetzung nach Anspruch 15, **dadurch gekennzeichnet, dass** das ergänzende Wachs eine Härte größer oder gleich 0,05 MPa und unter 6 MPa aufweist.

18. Zusammensetzung nach Anspruch 17, **dadurch gekennzeichnet, dass** das ergänzende Wachs unter Bienenwachs, hydriertem Ricinusöl, Lanolinwachs, Paraffinwachs und Bayberry-Wachs ausgewählt ist.

19. Zusammensetzung nach einem der Ansprüche 15 bis 18, **dadurch gekennzeichnet, dass** das ergänzende Wachs in einer Menge von 5 bis 35 Gew.-%, bezogen auf das Gesamtgewicht der Zusammensetzung, und vorzugsweise 15 bis 25 Gew.-% enthalten ist.

20. Zusammensetzung nach einem der vorhergehenden Ansprüche, **dadurch gekennzeichnet, dass** es sich bei der Zusammensetzung um eine Wasser-in-Öl-Emulsion handelt.

21. Zusammensetzung nach einem der vorhergehenden Ansprüche, **dadurch gekennzeichnet, dass** die Zusammensetzung ferner mindestens einen Zusatzstoff enthält, der unter den Verdickungsmitteln, Weichmachern, Füllstoffen, Farbmitteln, Konservierungsmitteln, Hydratisierungsmitteln, Parfums, Vitaminen, Alkalisierungsmitteln, Ansäuerungsmitteln und deren Gemischen ausgewählt ist.

22. Zusammensetzung nach einem der vorhergehenden Ansprüche, **dadurch gekennzeichnet, dass** die Zusammensetzung eine Zusammensetzung zum Überziehen der Wimpern ist.

23. Zusammensetzung nach einem der vorhergehenden Ansprüche, **dadurch gekennzeichnet, dass** es sich bei der Zusammensetzung um Mascara handelt.

24. Mascara-Produkt, das ein Reservoir mit einer Mascara-Zusammensetzung und ein System zum Aufbringen der Zusammensetzung auf die Keratinfasern umfasst, **dadurch gekennzeichnet, dass** die Zusammensetzung eine Zusammensetzung nach einem der Ansprüche 1 bis 23 ist.

25. Kosmetisches Verfahren zum Überziehen von Keratinfasern und insbesondere Wimpern, das das Aufbringen einer Zusammensetzung nach einem der Ansprüche 1 bis 23 auf die Keratinfasern umfasst.

26. Verwendung einer Zusammensetzung nach einem der Ansprüche 1 bis 23, um Keratinfasern und insbesondere Wimpern zu krümmen.

27. Verwendung einer Zusammensetzung nach einem der Ansprüche 4 bis 23, um Keratinfasern und insbesondere Wimpern zu verdicken und/oder die Keratinfasern füllig zu schminken.

28. Verwendung einer Zusammensetzung, die in einem physiologisch akzeptablen Medium eine wässrige Mikrodispersion von Wachspartikeln, die Wachspartikel mit einer Größe unter 1 µm enthält, wobei das Wachs eine Härte größer oder gleich 6 MPa und vorzugsweise im Bereich von 6 bis 15 MPa aufweist, und eine flüssige Fettphase enthält, die mindestens ein flüchtiges organisches Lösungsmittel aufweist, das bei Umgebungsdruck und Raumtemperatur einen Dampfdruck im Bereich von 10⁻³ bis 300 mm Hg (0,13 Pa bis 40 000 Pa) besitzt, mit der Maßgabe, dass die Siedetemperatur über 30 °C liegt, um Keratinfasern und insbesondere Wimpern, zu verdichten und/oder zu krümmen.

29. Verwendung einer wässrigen Mikrodispersion von Wachspartikeln, die Wachspartikel mit einer mittleren Größe unter 1 µm enthält, einer flüssigen Fettphase, die mindestens ein flüchtiges organisches Lösungsmittel aufweist, das bei Umgebungsdruck und Raumtemperatur einen Dampfdruck im Bereich von 10⁻³ bis 300 mm Hg (0,13 Pa bis 40 000 Pa) besitzt, mit der Maßgabe, dass die Siedetemperatur über 30 °C liegt, und eines ergänzenden Wachses, das in der flüssigen Fettphase vorliegt, in einer Zusammensetzung, die ein physiologisch akzeptables Medium enthält, um Keratinfasern und insbesondere Wimpern zu verdicken.

30. Verwendung einer wässrigen Mikrodispersion von Wachspartikeln, die Wachspartikel mit einer mittleren Größe unter 1 µm enthält, wobei das Wachs eine Härte größer oder gleich 6 MPa und vorzugsweise im Bereich von 6 bis 15 MPa aufweist, einer flüssigen Fettphase, die mindestens ein flüchtiges organisches Lösungsmittel aufweist, das bei Umgebungsdruck und Raumtemperatur einen Dampfdruck im Bereich von 10⁻³ bis 300 mm Hg (0,13 Pa bis 40 000 Pa) besitzt, mit der Maßgabe, dass die Siedetemperatur über 30 °C liegt, und eines ergänzenden Wachses, das in der flüssigen Fettphase vorliegt, in einer Zusammensetzung, die ein physiologisch akzeptables Medium enthält, um Keratinfasern und insbesondere Wimpern zu verdicken und/oder zu krümmen.
